# EUROPEAN PATENT APPLICATION

(11) **EP 3 763 808 A1**
(43) Date of publication of application: **13.01.2021**
(21) Application number: 19763434.8
(22) Date of filing: 08.03.2019
(51) Int. Cl.: C12M 1/00, C12M 1/28

(54) **PARTICLE SEPARATION DEVICE**

(30) Priority: 08.03.2018 JP 2018042205
(71) Applicant: Toyama Prefecture, Toyama 930-8501 (JP); Zeon Corporation, Tokyo 100-8246 (JP)
(72) Inventor: TAKATA, Kouji, Toyama-shi, Toyama 933-0866 (JP); HASHIOKA, Shingi, Tokyo 100-8246 (JP)
(74) Representative: Maiwald Patent- und Rechtsanwaltsgesellschaft mbH
(86) International application number: PCT/JP2019/009373
(87) International publication number: WO 2019/172428

(57) **Abstract**

[Object] To provide a particle separation device which is easy to be operated and is able to appropriately separate particles.

[Solving Means] A particle separation device includes: a syringe 2; a barrel 41 into which a sample liquid S containing target particles is injected; a barrel 51 into which a buffer liquid B is injected; a branching tube 3 which includes a first conduit 31 connected to a discharge port 21a of the syringe 2, a second conduit 32 corresponding to one of two branching from the first conduit 31 and connected to an injection port of the barrel 41, and a third conduit 33 corresponding to the other of two branching from the first conduit and connected to an injection port of the barrel 51; a first unidirectional valve 31a which is provided in the middle of the first conduit so as to prevent a backflow and is operated to be opened in a positive pressure state on the side of the syringe and to be closed in a negative pressure state on the side of the syringe; and a DLD microchannel chip 6 which has a DLD microchannel structure.

## Description

### TECHNICAL FIELD

The present invention relates to a particle separation device that separates target particles from a liquid containing the target particles.

### BACKGROUND ART

There is known that tumor cells are released from a primary tumor tissue or metastatic tumor tissue of cancer and infiltrate into blood. Such tumor cells are called circulating tumor cells (CTC) and are expected as information for diagnosing a progress status of cancer and therapeutic effect (prognosis) or for early detection of recurrence/metastasis by measuring the number of the circulating tumor cells in the peripheral blood.

As a method of measuring the number of the circulating tumor cells in the peripheral blood, for example, as described in Patent Document 1, there is known a method in which cells in a cell sample liquid is stained with fluorescently labeled antibody, the cell sample liquid is poured into a channel of a cell sorter chip, circulating tumor cells to be collected and other cells are separated using an image processing cell sorter, and the number of the separated circulating tumor cells is counted. However, since the image processing cell sorter essentially requires a camera for observing a flow in the cell sorter chip, an image recognition system for distinguishing and recognizing the cell types in an image captured by the camera, and a device for applying an external force (a voltage or the like) to cells so as to sort the differentiated cells and transfer them to different channels, there are problems that the image processing cell sorter is an expensive and large device and cannot be used simply.

As a method which can be more simply used to measure the number of the circulating tumor cells without using the device or the like including a mechanism having a complex structure such as a cell sorter, there is known a method using Deterministic Lateral Displacement (DLD) that uses a tendency that circulating tumor cells are larger than blood cells (for example, see Patent Document 2 and Non-Patent Document 1). As a separation device using this separation technique, Fig. 2 of Non-Patent Document 2 experimentally illustrates one in which a microchannel chip (substrate) designed based on a basic structure of a DLD method is provided with a sample liquid injection system including a syringe and a tube for injecting a sample liquid and a buffer liquid injection system including a syringe and a tube for injecting a buffer liquid.

However, in the separation device described in Non-Patent Document 2, since the sample liquid injection system and the buffer liquid injection system are independent from each other and are provided with the syringes and the tubes, the syringe into which the sample liquid is injected and the syringe into which the buffer liquid is injected need to be operated (the pistons need to be pushed) by the same amount at the same time. Accordingly, since an operation is complicated in that both hands need to be used for an operation performed alone and the flow rates of the sample liquid and the buffer liquid are largely different when both syringes are not carefully and equally operated, there is concern that an appropriate separation cannot be performed.

### CITATION LIST

### PATENT DOCUMENT

Patent Document 1: WO 2011/105507 A
Patent Document 2: US 7,735,652

### NON-PATENT DOCUMENT

Non-Patent Document 1: Huang et al., "Continuous Particle Separation Through Deterministic Lateral Displacement", Science 304, p. 987-990, 2004.
Non-Patent Document 2: Hiromasa Okano with 6 others, "First Screening of Circulating Tumor Cells in Blood by Dimensional Difference - The effect of cell hardness -", Proceedings of JSPE Spring Conference Academic Lecture 2014, The Japan Society for Precision Engineering, p. 453-454

### SUMMARY OF THE INVENTION

### PROBLEM TO BE SOLVED BY THE INVENTION

The invention has been made in view of such circumstances and an object of the invention is to provide a particle separation device which is easy to be operated and is able to appropriately separate particles.

### MEANS FOR SOLVING PROBLEM

In order to achieve the above-described object, a particle separation device according to a first aspect of the invention is a particle separation device for separating target particles from a sample liquid containing the target particles comprising: a syringe including a barrel having a discharge port and a piston for being pushed to extrude a gas inside the barrel from the discharge port by pushing the piston; a sample liquid storing member including an injection port and a discharge port for injecting and discharging the sample liquid; a buffer liquid storing member including an injection port and a discharge port for injecting and discharging a buffer liquid; a branching tube including a first conduit connected to the discharge port of the barrel, a second conduit being one of two branches from the first conduit and connected to the injection port of the sample liquid storing member, and a third conduit being the other of two branches from the first conduit and connected to the injection port of the buffer liquid storing member; a first unidirectional valve provided in the middle of the first conduit so as to prevent a backflow and operated to be opened in a positive pressure state on the side of the syringe and to be closed in a negative pressure state on the side of the syringe; and a DLD microchannel chip including, a sample liquid introduction port connected to the discharge port of the sample liquid storing member, a buffer liquid introduction port connected to the discharge port of the buffer liquid storing member, a DLD channel portion having a DLD microchannel structure provided with a plurality of fine pillars allowing the sample liquid introduced through the sample liquid introduction port and the buffer liquid introduced through the buffer liquid introduction port to flow in parallel in a contact state by pushing the piston, a buffer liquid discharge port discharging the buffer liquid containing target particles moved from the sample liquid to the buffer liquid in the DLD channel portion, and a sample liquid discharge port discharging the sample liquid after target particles moved to the buffer liquid in the DLD channel portion.

At the time of separating particles using the particle separation device according to the first aspect of the invention, when the piston of the syringe is pushed, a gas inside the barrel of the syringe is extruded and the internal pressure of the branching tube is increased, so that the pressure acts on the sample liquid storing member storing the sample liquid and the buffer liquid storing member storing the buffer liquid at the same time and the sample liquid and the buffer liquid are supplied to the corresponding introduction port of the DLD microchannel chip. Thus, since the syringe can be operated with one hand in that the syringe is a single member, the syringe can be easily operated and both the sample liquid and the buffer liquid can be caused to flow at an appropriate flow rate without a particularly careful operation, whereby an appropriate separation can be realized. Further, since the particle separation device according to the first aspect of the invention includes the first unidirectional valve for preventing a backflow in the middle of the first conduit, the syringe is not pushed back even when a hand is released from the piston after the internal pressure on the downstream side of the first unidirectional valve of the first conduit (the side of the second conduit and the side of the third conduit) is increased by pushing the piston of the syringe with a hand.

In the particle separation device according to the first aspect of the invention, the sample liquid can contain non-target particles different in size from the target particles. The particle separation device according to the first aspect of the invention can separate the target particles even when the sample liquid contains non-target particles different in size from the target particles.

In the particle separation device according to the first aspect of the invention, the sample liquid can be liquid containing cells as the target particles. In this case, the sample liquid can contain circulating tumor cells as the target particles. The particle separation device according to the first aspect of the invention can be appropriately used to separate cells such as circulating tumor cells.

In the particle separation device according to the first aspect of the invention, the second conduit can be attachable to and detachable from the injection port of the sample liquid storing member and the third conduit can be attachable to and detachable from the injection port of the buffer liquid storing member. With such a configuration, the second conduit can be attached to the injection port of the sample liquid storing member after the sample liquid is injected into the sample liquid storing member while the second conduit is detached from the injection port of the sample liquid storing member and the third conduit can be attached to the injection port of the buffer liquid storing member after the buffer liquid is injected into the buffer liquid storing member while the third conduit is detached from the injection port of the buffer liquid storing member.

The particle separation device according to the first aspect of the invention can further comprise an external air sucking ventilation port provided so as to penetrate a wall portion from the inside to the outside on the first conduit at the side of the syringe in relation to the first unidirectional valve of the first conduit and provided with a second unidirectional valve operated to be closed in a positive pressure state on the side of the syringe and to be opened in a negative pressure state on the side of the syringe. With such a configuration, since the pushed piston can be pulled back and can be pushed again without detaching the syringe from the particle separation device, the internal pressure on the downstream side of the first unidirectional valve of the first conduit (the side of the second conduit and the side of the third conduit) can be sequentially increased by pumping (pushing and pulling) the piston. Thus, since a large amount of the sample liquid and the buffer liquid can be smoothly supplied to the DLD microchannel chip even when one having a small capacity with a small barrel cross-sectional area is used as the syringe, it is possible to appropriately prevent an increase in the size of the device or deterioration in the operability of the syringe in accordance with the use of the syringe having a large capacity with a large barrel cross-sectional area.

In order to achieve the above-described object, a particle separation device according to a second aspect of the invention is a particle separation device for separating target particles from a sample liquid containing the target particles comprising: a pressure generating device including a chamber having a discharge port and a movable portion for being moved by a certain amount to extrudes a gas inside the chamber from the discharge port by a certain amount; a sample liquid storing member including an injection port and a discharge port for injecting and discharging the sample liquid; a buffer liquid storing member including an injection port and a discharge port for injecting and discharging a buffer liquid; a branching tube including a first conduit connected to the discharge port of the chamber, a second conduit being one of two branches from the first conduit and connected to the injection port of the sample liquid storing member, and a third conduit being to the other of two branches from the first conduit and connected to the injection port of the buffer liquid storing member; a first unidirectional valve provided in the middle of the first conduit or in the discharge port of the chamber so as to prevent a backflow and operated to be opened in a positive pressure state on the side of the pressure generating device and to be closed in a negative pressure state on the side of the pressure generating device; and a DLD microchannel chip including, a sample liquid introduction port connected to the discharge port of the sample liquid storing member, a buffer liquid introduction port connected to the discharge port of the buffer liquid storing member, a DLD channel portion having a DLD microchannel structure provided with a plurality of fine pillars allowing the sample liquid introduced through the sample liquid introduction port and the buffer liquid introduced through the buffer liquid introduction port to flow in parallel in a contact state in such a manner that the pressure generating device increases the pressure inside the sample liquid storing portion and the pressure inside the buffer liquid storing portion, a buffer liquid discharge port discharging the buffer liquid containing target particles moved from the sample liquid to the buffer liquid in the DLD channel portion, and a sample liquid discharge port discharging the sample liquid after target particles moved to the buffer liquid in the DLD channel portion.

At the time of separating particles using the particle separation device according to the second aspect of the invention, when the movable portion of the pressure generating device is moved by a certain amount, a gas inside the chamber of the pressure generating device is pushed by a certain amount and the internal pressure of the branching tube is increased, so that the pressure acts on the sample liquid storing member storing the sample liquid and the buffer liquid storing member storing the buffer liquid at the same time and the sample liquid and the buffer liquid are supplied to the corresponding introduction port of the DLD microchannel chip. Thus, since only the single pressure generating device may be provided, the device can be simplified when the device is driven by power such as electric power other than human power and both the sample liquid and the buffer liquid can be caused to flow at an appropriate flow rate without a particular consideration, whereby an appropriate separation can be realized. Further, since the particle separation device according to the second aspect of the invention includes the first unidirectional valve which is provided in the middle of the first conduit or in the discharge port of the chamber so as to prevent the backflow, the internal pressure on the downstream side of the first unidirectional valve (the side of the second conduit and the side of the third conduit) is easily maintained even when the movable portion is stopped after increasing the internal pressure on the downstream side of the first unidirectional valve (the side of the second conduit and the side of the third conduit) by moving the movable portion of the pressure generating device by a certain amount. As a result, since a compressed air tank or the like for maintaining a pressure does not need to be provided, the device can be simplified.

In the particle separation device according to the second aspect of the invention, the sample liquid can contain non-target particles different in size from the target particles. The particle separation device according to the second aspect of the invention can separate the target particles even when the sample liquid contains non-target particles different in size from the target particles.

In the particle separation device according to the second aspect of the invention, the sample liquid can be liquid containing cells as the target particles. In this case, the sample liquid can contain circulating tumor cells as the target particles. The particle separation device according to the second aspect of the invention can be appropriately used to separate cells such as circulating tumor cells.

In the particle separation device according to the second aspect of the invention, the second conduit can be attachable to and detachable from the injection port of the sample liquid storing member and the third conduit can be attachable to and detachable from the injection port of the buffer liquid storing member. With such a configuration, the second conduit can be attached to the injection port of the sample liquid storing member after the sample liquid is injected into the sample liquid storing member while the second conduit is detached from the injection port of the sample liquid storing member and the third conduit can be attached to the injection port of the buffer liquid storing member after the buffer liquid is injected into the buffer liquid storing member while the third conduit is detached from the injection port of the buffer liquid storing member.

The particle separation device according to the second aspect of the invention can further include an external air sucking ventilation port provided so as to penetrate a wall portion from the inside to the outside on the first conduit at the side of the pressure generating device in relation to the first unidirectional valve of the first conduit or provided so as to penetrate a wall portion of the chamber from the inside to the outside, and provided with a second unidirectional valve operated to be closed in a positive pressure state on the side of the pressure generating device and to be opened in a negative pressure state on the side of the pressure generating device. With such a configuration, since the movable portion inside the chamber can perform an operation of generating a positive pressure, perform an opposite operation (that is, an operation of generating a negative pressure), and then perform an operation of generating a positive pressure again without detaching the pressure generating device from the particle separation device, the internal pressure on the downstream side of the first unidirectional valve of the first conduit (the side of the second conduit and the side of the third conduit) can be sequentially increased by repeating an operation of generating a positive pressure. Thus, since a large amount of the sample liquid and the buffer liquid can be smoothly supplied to the DLD microchannel chip even when one having a small-capacity chamber is used as the pressure generating device, it is possible to appropriately prevent an increase in the size or an increase in the cost of the device in accordance with the use of the pressure generating device having many large-capacity chambers.

### BRIEF DESCRIPTION OF DRAWINGS

Fig. 1 is a front view schematically illustrating an overall configuration of a CTC separation device according to an embodiment of the invention;
Fig. 2 is a plan view illustrating a schematic configuration of a DLD microchannel chip of the CTC separation device of Fig. 1;
Fig. 3 is a diagram illustrating a particle separation principle of the DLD microchannel chip illustrated in Fig. 2; and
Fig. 4 is a front view schematically illustrating an overall configuration of a CTC separation device according to another embodiment of the invention.

### MODE(S) FOR CARRYING OUT THE INVENTION

Hereinafter, a CTC separation device for separating circulating tumor cells from a sample liquid corresponding to a blood derived liquid containing blood cells as non-target particles and circulating tumor cells (CTC) as target particles will be described with reference to the drawings as an embodiment of a particle separation device according to the invention for separating (fractionating) target particles from a sample liquid containing target particles and non-target particles different in size from the target particles.

However, the invention is not limited to one separating the circulating tumor cells and can be also applied to one separating cells correlated with target particles from a body liquid (including blood, lymph, saliva, urine, tears, and the like) containing cells other than circulating tumor cells as target particles and cells different in size from the target particles as non-target particles. Further, the target particles and the non-target particles are not limited to cells and the invention can be broadly applied to those separating particles correlated with target particles from a liquid in which two kinds of particles having different sizes are dispersed. Additionally, "dispersion" mentioned herein includes not only a case in which particles (cells) are suspended as single particles in a liquid, but also a case in which some or all of them are suspended as clusters. Further, this includes not only a case in which particles are scattered and floated in a liquid but also a case in which particles are settled to some extent. The particle diameters of the target particles and the non-target particles are about 0.1 to 1000 µm.

Further, in the invention, the sample liquid may be one containing target particles to be separated and non-target particles different in size from the target particles does not need to be essentially contained. For example, the invention can be also applied to a case in which a buffer liquid containing particles of a single size or a plurality of kinds of particles having a predetermined size or more is exchanged (a buffer liquid containing particles is used as a sample liquid and the particles are transferred to another buffer liquid having a different element or the same element as that of the sample liquid) or a case in which the concentration of particles is concentrated. Further, the "target particles" mean particles to be separated and also mean not only particles to be separated and collected for a certain usage (for example, inspection) but also particles which need to be separated and removed (eliminated) because they are not necessary.

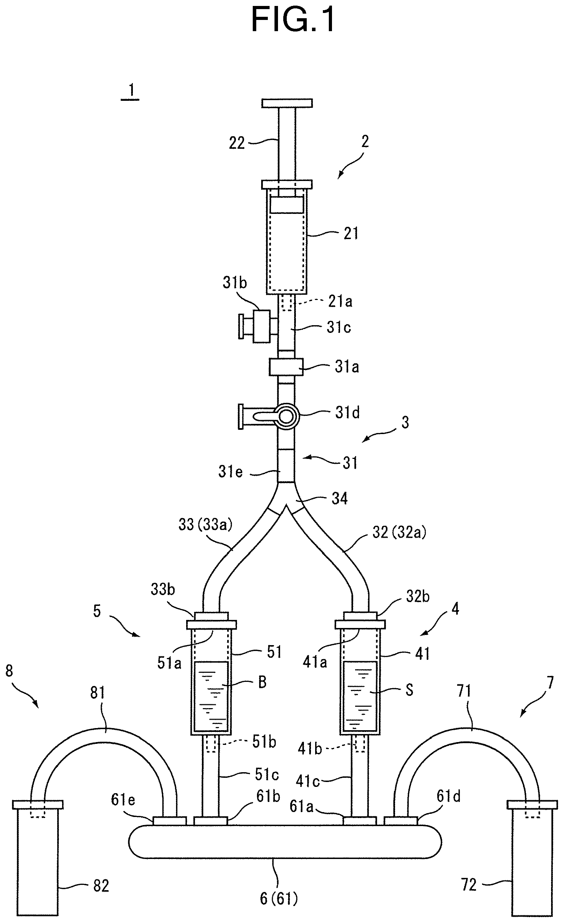

As illustrated in Fig. 1, a CTC separation device 1 according to the embodiment schematically includes a syringe 2 which is a pressure generating device, a pressure distributing portion (branching tube) 3, a sample liquid storing portion (sample liquid storing member) 4, a buffer liquid storing portion (buffer liquid storing member) 5, a separation portion (DLD microchannel chip) 6, a sample liquid collecting portion 7, and a buffer liquid collecting portion 8.

The syringe 2 is a piston syringe which includes a barrel (outer cylinder) 21 including a discharge port 21a and a piston 22 and is used to extrude (discharge) a gas (air) inside the barrel 21 from the discharge port 21a by pushing (sliding in a pushing direction) the piston 22 and to suck the gas from the discharge port 21a by sliding the piston in a pulling direction.

The pressure distributing portion 3 is used to distribute a pressure generated by the syringe 2 and includes a first conduit 31 which is connected to the discharge port 21a of the barrel 21 of the syringe 2, a second conduit 32 which is one of two branching from the first conduit 31 and is connected to an injection port 41a of a barrel 41 of the sample liquid storing portion 4 to be described later, and a third conduit 33 which is the other of two branching from the first conduit 31 and is connected to an injection port 51a of a barrel 51 of the buffer liquid storing portion 5 to be described later.

The first conduit 31 includes a unidirectional valve (first unidirectional valve) 31a, a unidirectional valve (second unidirectional valve) 31b, a T-shaped tube connector 31c, a three-way stopcock 31d, and a tube 31e.

The unidirectional valve 31a is provided in the middle of the first conduit 31 and the unidirectional valve 31a is a backflow preventing valve which is provided to be opened when an upstream side (a side of the syringe 2) is in a positive pressure state compared to a downstream side (a side of the second conduit 32 and a side of the third conduit 33) and to be closed in a negative pressure state compared to the downstream side. As the unidirectional valve 31a, a diaphragm type check valve can be used.

A ventilation port for sucking external air is provided on the side of the syringe 2 in relation to the unidirectional valve 31a of the first conduit 31. The ventilation port is provided so as to penetrate a wall portion of the first conduit 31 from the inside to the outside and includes the unidirectional valve (second unidirectional valve) 31b which is provided so as to be closed when a positive pressure is formed on the side of the syringe 2 and to be opened when a negative pressure is formed on the side of the syringe. As the unidirectional valve 31b, a diaphragm type check valve can be used similarly to the unidirectional valve 31a. In the embodiment, the ventilation port is realized by a configuration in which the T-shaped tube connector 31c including first to third connection ports is provided in the middle of the first conduit 31, that is, a configuration in which a first connection port of the T-shaped tube connector 31c is connected to the discharge port 21a of the barrel 21 of the syringe 2, a second connection port is connected to one connection port of the unidirectional valve 31a, a third connection port is connected to the other connection port of the unidirectional valve 31b, and the other connection port of the unidirectional valve 31b is opened to the outside.

The three-way stopcock 31d including first to third connection ports and a channel changing lever is provided on the downstream side of the unidirectional valve 31a of the first conduit 31 (the side of the sample liquid storing portion 4 and the side of the buffer liquid storing portion 5). More specifically, the first connection port of the three-way stopcock 31d is connected to the connection port on the downstream side of the unidirectional valve 31a, the tube 31e is connected to the second connection port, and the third connection port is exposed to the outside. When the lever of the three-way stopcock 31d is rotated in a predetermined direction, only the route from the first connection port to the second connection port can be opened, only the route from the first connection port to the third connection port can be opened, or only the route from the second connection port to the third connection port can be opened.

The second conduit 32 includes a tube 32a and an adapter 32b attached to one end of the tube 32a. The adapter 32b of the second conduit 32 is removably attached to the injection port 41a of the barrel 41 of the sample liquid storing portion 4 to be described later. The third conduit 33 includes a tube 33a and an adapter 33b attached to one end of the tube 33a. The adapter 33b of the third conduit 33 is removably attached to the injection port 51a of the barrel 51 of the buffer liquid storing portion 5 to be described later.

An end portion on the downstream side of the tube 31e of the first conduit 31 (on the side opposite to the three-way stopcock 31d) is connected to a first connection port of a Y-shaped tube connector 34 including first to third connection ports. An end portion on the upstream side of the tube 32a of the second conduit 32 (on the side opposite to the adapter 32b) is connected to the second connection port of the Y-shaped tube connector 34 and an end portion on the upstream side of the tube 33a of the third conduit 33 (on the side opposite to the adapter 33b) is connected to the third connection port of the Y-shaped tube connector 34.

The sample liquid storing portion 4 includes the barrel (a member corresponding to the outer cylinder of the syringe) 41 and the barrel 41 includes the injection port 41a and the discharge port 41b. The barrel 41 stores a sample liquid (blood) S containing target particles (circulating tumor cells) and non-target particles (blood cells) different in size from the target particles. Additionally, a diluted blood (for example, a blood diluted 2 times with PBS containing EDTA at a concentration of 4 mM) can be used as the sample liquid S.

The barrel 41 is supported by a stand or the like (not illustrated) while its longitudinal direction (axial direction) is set to be approximately vertical so that the injection port 41a is located at the upper side and the discharge port 41b is located at the lower side. The sample liquid S is injected while the adapter 32b of the second conduit 32 is detached from the injection port 41a of the barrel 41 and the adapter 32b of the second conduit 32 is air-tightly attached to the injection port 41a of the barrel 41 after the sample liquid is injected. The sample liquid S stored in the barrel 41 is discharged from the discharge port 41b when a pressure is applied through the second conduit 32.

The buffer liquid storing portion 5 includes the barrel (a member corresponding to the outer cylinder of the syringe) 51 and the barrel 51 includes the injection port 51a and the discharge port 51b. The barrel 51 stores a buffer liquid B. As the buffer liquid B, one kind or a mixture of plural kinds of isotonic solutions can be used and for example, PBS or glycerin-containing PBS can be used.

The barrel 51 is supported by a stand or the like (not illustrated) while its longitudinal direction (axial direction) is set to be approximately vertical so that the injection port 51a is located at the upper side and the discharge port 51b is located at the lower side similarly to the barrel 41. The buffer liquid B is injected, for example, by the same amount as the sample liquid S while the adapter 33b of the third conduit 33 is detached from the injection port 51a of the barrel 51 and the adapter 33b of the third conduit 33 is air-tightly attached to the injection port 51a of the barrel 51 after the buffer liquid is injected. The buffer liquid B stored in the barrel 51 is discharged from the discharge port 51b when a pressure is applied through the third conduit 33.

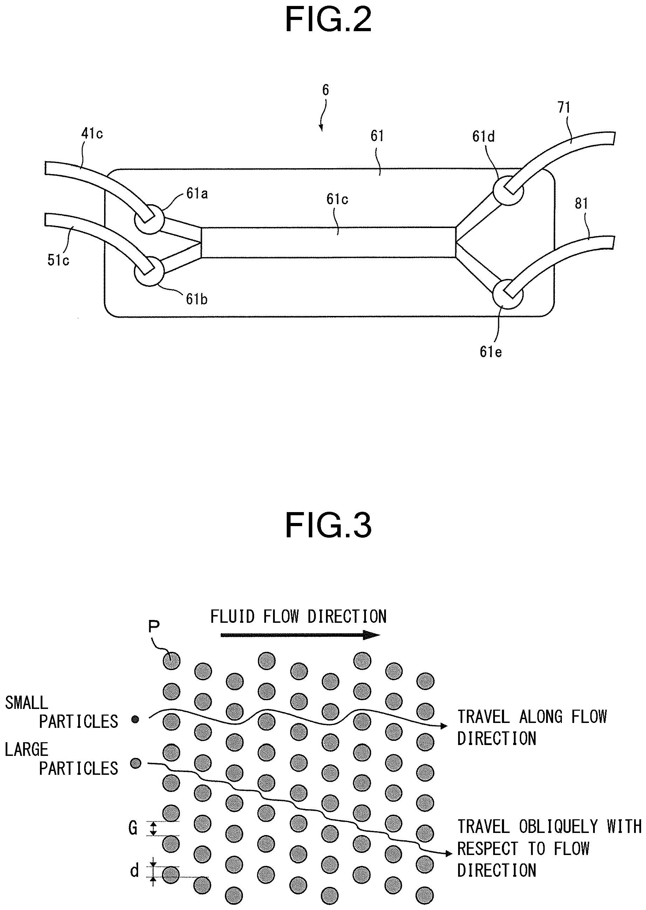

The separation portion 6 includes a DLD microchannel chip 61 and a chip holder (not illustrated) for preventing a leakage. The DLD microchannel chip 61 includes, also as illustrated in Fig. 2, a sample liquid introduction port 61a, a buffer liquid introduction port 61b, a DLD channel portion 61c, a sample liquid discharge port 61d, and a buffer liquid discharge port 61e.

The discharge port 41b of the barrel 41 of the sample liquid storing portion 4 is connected to the sample liquid introduction port 61a through the tube 41c. The discharge port 51b of the barrel 51 of the buffer liquid storing portion 5 is connected to the buffer liquid introduction port 61b through the tube 51c. The DLD channel portion 61c has a DLD microchannel structure in which a plurality of fine pillars are arranged. Additionally, the DLD channel portion 61c, the route from the discharge port 41b of the barrel 41 of the sample liquid storing portion 4 to the DLD channel portion 61c (the channel including the discharge port 41b, the tube 41c, the sample liquid introduction port 61a, and the DLD channel portion 61c), and the route from the discharge port 51b of the barrel 51 of the buffer liquid storing portion 5 to the DLD channel portion 61c (the channel including the discharge port 51b, the tube 51c, the buffer liquid introduction port 61b, and the DLD channel portion 61c) are preferably filled with a buffer liquid (PBS or glycerin-containing PBS) in advance.

Due to the pressure supplied through the piston 22 of the syringe 2, the sample liquid S inside the barrel 41 of the sample liquid storing portion 4 is introduced into the DLD channel portion 61c through the discharge port 41b, the tube 41c, and the sample liquid introduction port 61a and the buffer liquid B inside the barrel 51 of the buffer liquid storing portion 5 is introduced into the DLD channel portion 61c through the discharge port 51b, the tube 51c, and the buffer liquid introduction port 61b.

The sample liquid introduced through the sample liquid introduction port 61a and the buffer liquid introduced through the buffer liquid introduction port 61b flow through the DLD channel portion 61c in parallel as a laminar flow while being in contact with each other. The DLD channel portion 61c has, for example, a plurality of fine pillars (micropillars) arranged according to the principle of the deterministic lateral displacement (DLD) method as described in Non-Patent Document 1.

The deterministic lateral displacement method is, as illustrated in Fig. 3, a separation method that utilizes a property in which small particles travel along a flow direction and large particles travel obliquely with respect to the flow direction since the traveling of large particles along the flow direction is disturbed by the existence of pillars when a particle dispersion liquid is caused to flow to a pillar group including a plurality of pillars P arranged according to a predetermined rule. By appropriately setting a threshold value determined by a gap G between the pillars P and a shift amount d thereof, particles having a diameter smaller than the threshold value and particles having a diameter equal to or larger than the threshold value can be separated.

Inside the DLD channel portion 61c, CTC (for example, about 12 µm) having a relatively large diameter and contained in the sample liquid travels obliquely with respect to the flow direction of the sample liquid and moves to the buffer liquid B flowing in parallel as a laminar flow while contacting the sample liquid so that the buffer liquid containing CTC moved from the sample liquid to the buffer liquid reaches the buffer liquid discharge port 61e. Inside the DLD channel portion 61c, blood cells (for example, about 8 µm) having a relatively small diameter and contained in the sample liquid travel along the flow direction of the sample liquid and reach the sample liquid discharge port 61d together with the sample liquid after CTC is moved (separated). Additionally, since the sample liquid and the buffer liquid are slightly mixed with each other when flowing in parallel as a laminar flow in a contact state inside the DLD channel portion 61c, a part of the buffer liquid may be contained in the sample liquid discharged from the sample liquid discharge port 61d and a part of the sample liquid may be contained in the buffer liquid discharged from the buffer liquid discharge port 61e.

A sample liquid collecting container 72 is connected to the sample liquid discharge port 61d through a tube 71 of the sample liquid collecting portion 7 and the sample liquid from which CTC is separated is collected by the sample liquid collecting container 72. A buffer liquid collecting container 82 is connected to the buffer liquid discharge port 61e through a tube 81 of the buffer liquid collecting portion 8 and the buffer liquid containing CTC moved (separated) from the sample liquid is collected by the buffer liquid collecting container 82.

At the time of separating CTC from a subject's blood, when the piston 22 of the syringe 2 is pushed, air inside the barrel 21 of the syringe 2 is extruded and the internal pressure of the pressure distributing portion 3 (the first conduit 31, the second conduit 32, and the third conduit 33) is increased, so that the pressure acts on the barrel 41 of the sample liquid storing portion 4 storing the sample liquid S and the barrel 51 of the buffer liquid storing portion 5 storing the buffer liquid B at the same time and the sample liquid S and the buffer liquid B are pressure-fed to the corresponding introduction ports 61a and 61b of the DLD microchannel chip 61 of the separation portion 6. Thus, since the syringe 2 can be operated with one hand in that the syringe 2 is a single member, the syringe can be easily operated and both the sample liquid S and the buffer liquid B can be caused to appropriately flow to the DLD microchannel chip 61 at the same flow rate without a particularly careful operation, whereby an appropriate separation can be realized.

Further, in the embodiment, since the unidirectional valve 31a for preventing a backflow is provided in the middle of the first conduit 31 so as to be opened in a positive pressure state on the side of the syringe 2 and to be closed in a negative pressure state on the side of the syringe, it is possible to prevent a backflow of extruded air by pushing the piston 22 of the syringe 2. Thus, since a hand can be released from the piston 22 if necessary after the piston 22 of the syringe 2 is pushed to increase the internal pressure on the downstream side of the unidirectional valve 31a of the first conduit 31 (the side of the second conduit 32 and the side of the third conduit 33), an operation for supplying a pressure is easy.

Additionally, in the embodiment, since the external air sucking ventilation port including the T-shaped tube connector 31c and the unidirectional valve 31b is provided on the side of the syringe 2 in relation to the unidirectional valve 31a of the first conduit 31 in addition to the unidirectional valve 31a, the piston 22 which is pushed once can be pulled back and pushed again while the syringe 2 is connected to the T-shaped tube connector 31c in corporation with the unidirectional valve 31a. Thus, the internal pressure on the downstream side of the unidirectional valve 31a of the first conduit 31 can be sequentially increased by pumping the syringe 2 (pushing and pulling the piston 22). For this reason, since a large amount of the sample liquid and the buffer liquid can be smoothly supplied to the DLD microchannel chip 61 even when one having a small capacity with a small barrel cross-sectional area is used as the syringe 2, it is possible to appropriately prevent an increase in the size of the device or deterioration in the operability of the syringe at the time of using the syringe having a large capacity with a large barrel cross-sectional area.

Additionally, in the embodiment, the second conduit 32 is attachable to and detachable from the injection port 41a of the barrel 41 so that the sample liquid is injected into the barrel 41 of the sample liquid storing portion 4 and the third conduit 33 is attachable to and detachable from the injection port 51a of the barrel 51 so that the buffer liquid is injected into the barrel 51 of the buffer liquid storing portion 5. However, these may be injected by other means without making them removable or by making them removable. For example, the three-way stopcock may be provided in the middle of each of the second conduit 32 and the third conduit 33 and the route of the three-way stopcock may be switched so that the sample liquid or the buffer liquid is injected into the corresponding barrel 41 or 51.

Further, in Fig. 1, the barrel 21 of the syringe 2 is depicted so as to be installed upright in a substantially vertical direction similarly to the barrel 41 of the sample liquid storing portion 4 and the barrel 51 of the buffer liquid storing portion 5, but the invention is not limited thereto. For example, a part or all of the first conduit 31, the second conduit 32, and the third conduit 33 may be made of a flexible material and the flexible portion may be bent so that the side of the syringe 2 hangs down by gravity.

Further, in the above-described embodiment, the syringe 2 is used as a pressure generating device corresponding to a pressure generating source for allowing the sample liquid and the buffer liquid to flow through the DLD microchannel chip 61. However, if the chamber including the discharge port and the movable portion can be provided and the movable portion can be moved by a certain amount so that a gas inside the chamber is extruded from the discharge port by a certain amount, other pressure generating devices may be used or a pressure generating device driven by power such as electric power other than human power may be used. Specific examples of other pressure generating devices include a pressure generating device including a chamber and a movable portion employed in an electric pump such as an electric rotary pump including a rotor as a movable portion, an electric diaphragm pump including a diaphragm as a movable portion, an electric plunger pump including a plunger as a movable portion, and an electric piston pump including a piston as a movable portion, but the invention is not limited to these. Additionally, it can be said that the syringe 2 of the above-described embodiment is the pressure generating device which includes the barrel 21 as the chamber and the piston 22 as the movable portion for changing the internal pressure of the chamber (the barrel 21).

In a case in which the pressure generating device other than the syringe 2 is used, the unidirectional valve (the first unidirectional valve) for preventing the backflow may be provided in the middle of the first conduit 31 similarly to the unidirectional valve 31a of the embodiment using the syringe 2, but the pressure generating device in which the unidirectional valve for preventing the backflow is provided in advance in the discharge port of the chamber (the route from the camber to the first conduit 31 in the pressure generating device) may be used so that the unidirectional valve is used as the first unidirectional valve. When the first unidirectional valve for preventing the backflow is provided, the internal pressure on the downstream side of the first unidirectional valve (the side of the second conduit and the side of the third conduit) is easily maintained even when the movable portion is stopped after increasing the internal pressure on the downstream side of the first unidirectional valve (the side of the second conduit and the side of the third conduit) by moving the movable portion of the pressure generating device by a certain amount. As a result, since a compressed air tank or the like for maintaining a pressure does not need to be provided, the device can be simplified.

Further, the external air sucking ventilation port including the second unidirectional valve may be provided in the wall portion of the first conduit 31 similarly to the unidirectional valve 31b of the embodiment using the syringe 2, but the pressure generating device in which the external air sucking ventilation port including the unidirectional valve provided in advance so as to penetrate the wall portion of the chamber itself of the pressure generating device may be used so that the unidirectional valve is used as the second unidirectional valve. When the external air sucking ventilation port including the second unidirectional valve is provided, the movable portion inside the chamber can perform an operation of generating a positive pressure, perform an opposite operation (that is, an operation of generating a negative pressure), and then perform an operation of generating a positive pressure again without detaching the pressure generating device from the particle separation device. By repeating the operation for generating a positive pressure, the internal pressure on the downstream side of the first unidirectional valve of the first conduit (the side of the second conduit and the side of the third conduit) can be sequentially increased. Thus, since a large amount of the sample liquid and the buffer liquid can be smoothly supplied to the DLD microchannel chip even when one having a small-capacity chamber is used as the pressure generating device, it is possible to appropriately prevent an increase in the size or an increase in the cost of the device in accordance with the use of the pressure generating device including the chamber having a large capacity. Additionally, in a case in which the mechanism of the electric rotary pump is used, since a space on the side of the discharge port inside the chamber and a space on the side of the external air sucking ventilation port inside the chamber can be divided by a vane or the like so that a gas inside the chamber is not discharged from the external air sucking ventilation port, the external air sucking ventilation port without the second unidirectional valve can be provided.

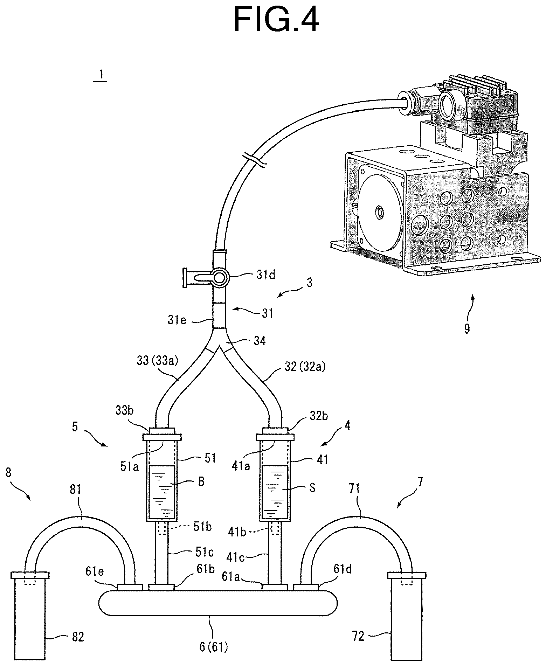

Fig. 4 illustrates a CTC separation device including an electric rotary pump 9 as a pressure generating device according to another embodiment of the invention. In the embodiment illustrated in Fig. 4, a unidirectional valve (not illustrated) for preventing a backflow is provided in the discharge port of the electric rotary pump 9 (the route from the chamber inside the electric rotary pump 9 to the first conduit).

The above-described embodiments are described to facilitate the understanding of the invention and are not described to limit the invention. Therefore, each component disclosed in the above-described embodiments is intended to include all design changes and equivalents within the technical scope of the invention.

### EXPLANATIONS OF LETTERS OR NUMERALS

1 CTC SEPARATION DEVICE (PARTICLE SEPARATION DEVICE)
2 SYRINGE (PRES SURE GENERATING DEVICE)
21 BARREL (CHAMBER)
21a DISCHARGE PORT
22 PISTON (MOVABLE PORTION)
3 PRESSURE DISTRIBUTING PORTION (BRANCHING TUBE)
31 FIRST CONDUIT
31a UNIDIRECTIONAL VALVE (FIRST UNIDIRECTIONAL VALVE)
31b UNIDIRECTIONAL VALVE (SECOND UNIDIRECTIONAL VALVE, VENTILATION PORT)
31c T-SHAPED TUBE CONNECTOR (VENTILATION PORT)
31d THREE-WAY STOPCOCK
32 SECOND CONDUIT
32a TUBE
32b ADAPTER
33 THIRD CONDUIT
33a TUBE
33b ADAPTER
34 Y-SHAPED TUBE CONNECTOR
4 SAMPLE LIQUID STORING PORTION (SAMPLE LIQUID STORING MEMBER)
41 BARREL
41a INJECTION PORT
41b DISCHARGE PORT
41c TUBE
5 BUFFER LIQUID STORING PORTION (BUFFER LIQUID STORING MEMBER)
51 BARREL
51a INJECTION PORT
51b DISCHARGE PORT
51c TUBE
6 SEPARATION PORTION
61 DLD MICROCHANNEL CHIP
61a SAMPLE LIQUID INTRODUCTION PORT
61b BUFFER LIQUID INTRODUCTION PORT
61c DLD CHANNEL PORTION
61d SAMPLE LIQUID DISCHARGE PORT
61e BUFFER LIQUID DISCHARGE PORT
7 SAMPLE LIQUID COLLECTING PORTION
71 TUBE
72 SAMPLE LIQUID COLLECTING CONTAINER
8 BUFFER LIQUID COLLECTING PORTION
81 TUBE
82 BUFFER LIQUID COLLECTING CONTAINER
9 ELECTRIC ROTARY PUMP (PRESSURE GENERATING DEVICE)
B BUFFER LIQUID
P PILLAR
S SAMPLE LIQUID

## Claims

1. A particle separation device for separating target particles from a sample liquid containing the target particles comprising:
a syringe including a barrel having a discharge port and a piston for being pushed to extrude a gas inside the barrel from the discharge port by pushing the piston;
a sample liquid storing member including an injection port and a discharge port for injecting and discharging the sample liquid;
a buffer liquid storing member including an injection port and a discharge port for injecting and discharging a buffer liquid;
a branching tube including a first conduit connected to the discharge port of the barrel, a second conduit being one of two branches from the first conduit and connected to the injection port of the sample liquid storing member, and a third conduit being the other of two branches from the first conduit and connected to the injection port of the buffer liquid storing member;
a first unidirectional valve provided in the middle of the first conduit so as to prevent a backflow and operated to be opened in a positive pressure state on the side of the syringe and to be closed in a negative pressure state on the side of the syringe; and
a DLD microchannel chip including,
a sample liquid introduction port connected to the discharge port of the sample liquid storing member,
a buffer liquid introduction port connected to the discharge port of the buffer liquid storing member,
a DLD channel portion having a DLD microchannel structure provided with a plurality of fine pillars allowing the sample liquid introduced through the sample liquid introduction port and the buffer liquid introduced through the buffer liquid introduction port to flow in parallel in a contact state by pushing the piston,
a buffer liquid discharge port discharging the buffer liquid containing target particles moved from the sample liquid to the buffer liquid in the DLD channel portion, and
a sample liquid discharge port discharging the sample liquid after target particles moved to the buffer liquid in the DLD channel portion.

2. The particle separation device according to claim 1,
wherein the sample liquid contains non-target particles different in size from the target particles.

3. The particle separation device according to claim 1 or 2,
wherein the target particles are cells.

4. The particle separation device according to claim 3,
wherein the sample liquid contains circulating tumor cells as the target particles.

5. The particle separation device according to any one of claims 1 to 4,
wherein the second conduit is attachable to and detachable from the injection port of the sample liquid storing member and the third conduit is attachable to and detachable from the injection port of the buffer liquid storing member.

6. The particle separation device according to any one of claims 1 to 5, further comprising:
an external air sucking ventilation port provided so as to penetrate a wall portion from the inside to the outside on the first conduit at the side of the syringe in relation to the first unidirectional valve of the first conduit and provided with a second unidirectional valve operated to be closed in a positive pressure state on the side of the syringe and to be opened in a negative pressure state on the side of the syringe.

7. A particle separation device for separating target particles from a sample liquid containing the target particles comprising:
a pressure generating device including a chamber having a discharge port and a movable portion for being moved by a certain amount to extrudes a gas inside the chamber from the discharge port by a certain amount;
a sample liquid storing member including an injection port and a discharge port for injecting and discharging the sample liquid;
a buffer liquid storing member including an injection port and a discharge port for injecting and discharging a buffer liquid;
a branching tube including a first conduit connected to the discharge port of the chamber, a second conduit being one of two branches from the first conduit and connected to the injection port of the sample liquid storing member, and a third conduit being to the other of two branches from the first conduit and connected to the injection port of the buffer liquid storing member;
a first unidirectional valve provided in the middle of the first conduit or in the discharge port of the chamber so as to prevent a backflow and operated to be opened in a positive pressure state on the side of the pressure generating device and to be closed in a negative pressure state on the side of the pressure generating device; and
a DLD microchannel chip including,
a sample liquid introduction port connected to the discharge port of the sample liquid storing member,
a buffer liquid introduction port connected to the discharge port of the buffer liquid storing member,
a DLD channel portion having a DLD microchannel structure provided with a plurality of fine pillars allowing the sample liquid introduced through the sample liquid introduction port and the buffer liquid introduced through the buffer liquid introduction port to flow in parallel in a contact state in such a manner that the pressure generating device increases the pressure inside the sample liquid storing portion and the pressure inside the buffer liquid storing portion,
a buffer liquid discharge port discharging the buffer liquid containing target particles moved from the sample liquid to the buffer liquid in the DLD channel portion, and
a sample liquid discharge port discharging the sample liquid after target particles moved to the buffer liquid in the DLD channel portion.

8. The particle separation device according to claim 7,
wherein the sample liquid contains non-target particles different in size from the target particles.

9. The particle separation device according to claim 7 or 8,
wherein the target particles are cells.

10. The particle separation device according to claim 9,
wherein the sample liquid contains circulating tumor cells as the target particles.

11. The particle separation device according to any one of claims 7 to 10,
wherein the second conduit is attachable to and detachable from the injection port of the sample liquid storing member and the third conduit is attachable to and detachable from the injection port of the buffer liquid storing member.

12. The particle separation device according to any one of claims 7 to 11, further comprising:
an external air sucking ventilation port provided so as to penetrate a wall portion from the inside to the outside on the first conduit at the side of the pressure generating device in relation to the first unidirectional valve of the first conduit or provided so as to penetrate a wall portion of the chamber from the inside to the outside, and provided with a second unidirectional valve operated to be closed in a positive pressure state on the side of the pressure generating device and to be opened in a negative pressure state on the side of the pressure generating device.
